# EUROPEAN PATENT SPECIFICATION

(11) **EP 0 676 938 B1**
(45) Date of publication and mention of the grant of the patent: **21.07.1999**
(21) Application number: 92906557.1
(22) Date of filing: 16.03.1992
(51) Int. Cl.: A61F 2/08

(54) **IMPLANTABLE FIXING DEVICE FOR KNEE LIGAMENT PROSTHESIS**
IMPLANTIERBARE KNIEBANDPROTHESEN-FIXIERUNGSVORRICHTUNG
DISPOSITIF DE FIXATION IMPLANTABLE POUR PROTHESE DES LIGAMENTS DU GENOU

(30) Priority: 21.03.1991 GB 91059576
(43) Date of publication of application: 18.10.1995
(73) Proprietor: Seedhom, Bahaa Botros, Leeds West Yorkshire LS2 9HD (GB)
(72) Inventor: SEEDHOM, Bahaa, Botros, Leeds LS2 9HD (GB); COLLINS, Simon, Barnsley S75 6AG (GB)
(74) Representative: Orr, William McLean
(86) International application number: GB9200469
(87) International publication number: WO9216167

(56) References cited:
- DE-U- 9 002 844
- FR-A- 2 395 012
- FR-A- 2 590 792

## Description

This invention relates to an implantable fixing device for securing one end of an elongate flexible tensile element, such as a prosthetic ligament, and to a prosthetic ligament fixation system.

Prosthetic ligaments are being installed in patients to an ever increasing extend, and in one known form comprises a woven structure of implantable material which can be used to join the adjacent ends of a bone joint e.g. the femur to a tibia of a knee joint following ACL replacement surgery.

The prosthetic ligament joins the bones together, and is usually anchored at one end of a reamed-out bone tunnel by a suitable fixing device, and at its other end by a bone staple. One known type of prosthetic ligament is disclosed in more detail in EP 126520, and has a pouch near one end in which a bone plug can be introduced, and which anchors this end of the ligament at the entrance to the bone tunnel, and with the ligament being threaded through the bone tunnels formed in the femur and the tibia, and having its free end anchored by a bone staple driven into a suitable bone site and thereby trapping the ligament end.

The use of a woven prosthetic ligament has the advantage of providing a durable implantable tensile element with a long service life, and which allows tissue ingrowth over a period of time to improve the anchorage of the ends of the ligaments.

However, prosthetic ligaments do not need to be made of synthetic i.e. man made material, and there is also use of prosthetic ligaments buit-up from naturally available material (autogenous tissue) which may be "harvested" from a patient undergoing surgery which involves the need for a replacement ligament.

Semi-tendinosus or gracilis tissue can be used for this purpose, which is formed into a prosthetic ligament bundle looped at one end around a fixing or anchoring device, and the use of this procedure is particularly favoured by US surgeons.

FR-A- 2 395 012 discloses a prosthetic ligament fixation system comprising a prosthetic ligament which can be taken through first and second bone tunnels formed in adjoining bones of a bone joint and a first fixing device.

The first fixing device is suitable for holding one end of the ligament at or near to one end of the first bone tunnel and is intended to seat itself on to the surface of the bone surrounding an entrance to the end of the tunnel.

The first fixing device comprises a seating device having an annular bearing surface to seat on the surrounding surface at the entrance to the first bone tunnel and a shaft adjustably mounted on and extending away from the seating device to be located in the first bone tunnel. The shaft serves to hold one end of the ligament and is capable of adjusting its position relative to the seating device so that the seating device can apply substantially uniform pressure via its annular bearing surface while the ligament can extend along the bone tunnels without coming into engagement with the walls of the tunnels.

In one aspect, the present invention is concerned with a novel fixing divice for securing one end of an implantable flexible tensile element, such as a prosthetic ligament of any suitable material, which will include an implantable woven ligament and also naturally available material which can be formed into a prosthetic ligament, as well as other flexible tensile implantable elements, such as a suture.

In a further aspect, the invention is concerned with a prosthetic ligament fixation system.

In ACL replacement surgery, usually the upper end of the prosthetic ligament is anchored near the upper end of a bone tunnel reamed out of the femur, whereas the lower end of the ligament extends beyond the lower end of the tunnel reamed out of the tibia and this lower projecting end is anchored by a bone staple driven into a suitable site on the tibia with the end of the ligament being trapped under the bar or cross piece of the staple.

In order to secure the upper end of the ligament reliably in position, it is important to provide a fixing device which can bear on the surface of the bone surrounding the entrance to the bone tunnel formed in the femur, as this has the required strength and wear resistance to withstand the loads which will be applied to the fixing device by the ligament during articulation of the knee joint.

However, the surrounding surface of the bone is not always uniformly smooth, and neither is it always truly perpendicular to the general axis of the bone tunnel. This gives rise to a problem, in that the ligament has to be looped around a part of the fixing device, and then is able to apply a tensile force to the fixing device in a direction generally along the axis of the tunnel, which can cause improper seating of the fixing device on the surface of the bone surrounding the entrance to the tunnel, which can give rise to non-uniform bearing pressure, or to localised high pressure contact areas. Clearly, this is undesirable, as it may be the cause of unreliability and also may result in accelerated wear of the bone surface and ultimately in slippage of the fixing device with resulting loss of tension in the ligament.

The present invention therefore addresses this problem and seeks to provide an improved seating of the fixing device on the surrounding bone surface while permitting satisfactory attachment of the ligament around a part of the fixing device.

According to an aspect of the invention there is provided an implantable fixing device for securing one end of a flexible elongate element such as a prosthetic ligament which is taken through first and second bone tunnels formed in adjoining bones of a bone joint said fixing device being intended to seat itself on the surface of the bone surrounding an entrance to one of the bone tunnels and said device comprising:
a seating device comprising a collar which is shaped so as to seat at least partly on said surrounding bone surface; and
a cradle which is pivotally mounted on said collar, said cradle being U shaped having a base around which the ligament can be looped and a pair of parallel legs connected to the base and which are pivotally mounted at their free ends on said collar;
   wherein the cradle extends away from the seating device to be located in the first bone, said cradle serving to hold one end of the ligament and being capable of adjusting its position relative to the seating device, if necessary, so that the seating device can apply substantially uniform bearing pressure via an annular bearing surface while the ligament can extend along the bone tunnels without coming into engagement with the walls of the tunnels.

The free ends of the legs may have lugs which can seat each on a respective one of a pair of diametrically opposed seating recesses formed in the collar, and preferably these lugs are out-turned lugs. This therefore allows the U-shaped cradle to be mounted on the collar by inward deformation of the free ends of the legs, or by relative slidable movement of the base and legs through the collar until the lugs become seated on the respective seating recesses.

In a particularly preferred arrangement, a plug can be positioned within the cradle to occupy at least part of the entrance portion of the first bone tunnel, and preferably the plug is a bone plug derived from the bone material reamed out during formation of the bone tunnels, and preferably shaped so as to be able to fit within and to engage with the cradle, but also to have at least part of its outer surface located closely adjacent to the inner wall of the adjacent part of the bone tunnel. This will promote boney ingrowth from the wall of the tunnel to join with the bone plug and the cradle over a period of time, to further improve the anchoring of the elongate element e.g a ligament.

The end of the ligament remote from the cradle and seating device is preferably anchored in position by use of a fastening staple or bone staple, which can be driven into the bone near the exit of the ligament from the second bone tunnel.

The prosthetic ligament may be manufactured from woven implantable material, or may be derived from autogenous tissue harvested from the patient. Alternatively, the elongate implantable element may comprise a suture e.g of the type used with patellar tendon bone block grafts.

One embodiment of implantable fixing device, and of prosthetic ligament fixation system, according to the invention will now be described in detail, by way of example only, with reference to the accompanying drawings in which:
Figure 1 is a schematic illustration of an implantable fixing device being used to anchor the upper end of a prosthetic ligament derived from autogenous tissue and used in ACL replacement surgery:
Figures 2a and 2b are, respectively, a plan view and a side view of a collar of the fixing device shown in Figure 1; and.
Figure 3 is a side view of a cradle which inter-fits with the collar shown in Figure 2 in order to complete the assembly of the fixing device.

Referring now the drawings, there will be described, by way of example only, the use of a fixing device to secure the upper end of a prosthetic ligament derived from autogenous tissue and used in ACL replacement surgery. This is merely one example of a type of implantable flexible elongate tensile element with which the fixing device of the invention may be used. This example also comprises an embodiment of prosthetic ligament fixation system according to the invention.

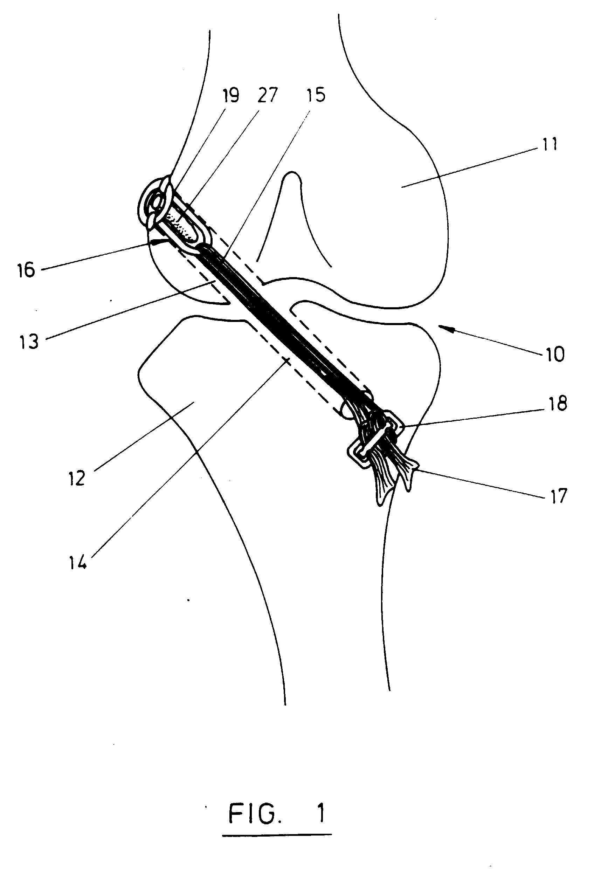

Referring particularly to Figure 1, this shows a typical knee joint 10 comprising a femur 11 and tibia 12, and through which have been formed, by reaming-out, aligned bone tunnels 13 and 14 respectively, for ACL replacement surgery. A prosthetic ligament 15 is shown extending through the tunnels 13 and 14, and is derived from autogenous tissue e.g. semi-tendinosus/gracilis material . The upper end of the prosthetic ligament 15 is looped around a fixing device according to the invention which is designated generally by reference 16, whereas the lower end 17 of the ligament projects beyond the lower end of bone tunnel 14 and can be anchored in position by a bone staple 18 e.g. of the type disclosed in WO 91/06249 which is driven into a suitable site in the tibia 12, thereby trapping the ligament end 17 securely.

The walls of the bone tunnels 13 and 14 are relatively soft, and therefore to secure reliably the upper end of the prosthetic ligament, it is important to provide a fixing device 16 which can seat itself reliably upon the bone surface surrounding the entrance 19 to the bone tunnel 13, which is sufficiently strong and wear resistant

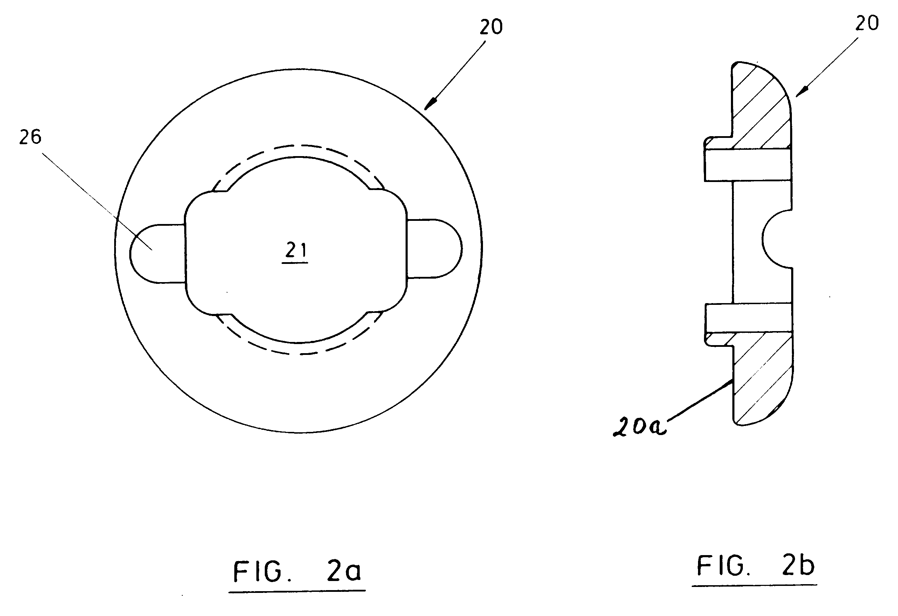

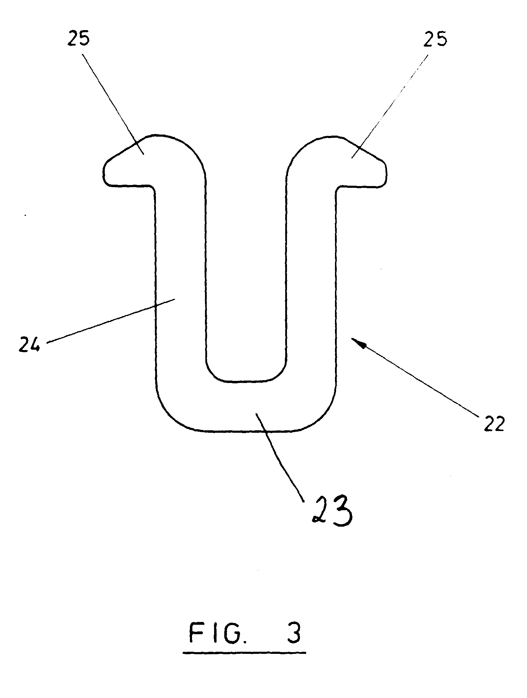

The construction of fixing device 16 is shown in more detail in Figures 2 and 3, and comprises a seating device in the form of a collar 20 which is shaped so as to seat on at least part of the annular bone surface surrounding the entrance 19 to bone tunnel 13 (via annular bearing surface 20a on its underside), and which has a through-hole 21 formed in the collar 20.

The fixing device 16 also includes a U-shaped cradle or stirrup 22 which is capable of being taken through the hole 21 in the collar 20 upon assembly, cradle 22 having a base 23 around which prosthetic ligament 15 can be taken, a pair of parallel legs 24 connected to the base 23, and a pair of out-turned lugs 25 on the free ends of the legs 24.

The lugs 25 are preferably out-turned and can seat each on a respective one of a pair of diametrically opposed seating recesses 26 formed in the collar 20 in order to complete the assembly, and provide a pivotally adjustable mounting of the cradle 22 on collar 20.

The collar 20 therefore can seat itself reliably and with substantially uniform bearing pressure upon the surrounding bone surface, and the cradle 22 can pivotally adjust itself, as necessary, via rocking engagement of lugs 25 on the seating recesses 26 so that the ligament 15 can extend along the bone tunnels 13 and 14 without snagging or other engagement with the walls of the tunnels.

The collar 20 is annular, and defines a circular hole through which the legs 24 of the cradle 22 can be taken upon assembly, until the lugs 25 come into engagement with the seating recesses 26. Alternatively, the legs 24 can be inwardly deformed to allow the ends of the legs 24 to pass through the collar, and then release of the deformation allows lugs 25 to snap-out into engagement with the recesses 26.

As can be seen from Figure 1, a bone plug 27 can complete the fixing device, and which is introduced through the hole 21 and which fits generally within the confines of the legs 24 and the base 23 of the cradle 22. The bone plug conveniently is fashioned from the bone material reamed-out during formation of the bone tunnels 13 and 14, and preferably is shaped so as to be able to fit within and to engage with the cradle, but also to have at least part of its outer surface closely adjacent to the wall of bone tunnel 13. This will promote boney ingrowth from the wall of the tunnel to join with the bone plug and the cradle over a period of time, to further improve the anchoring of the ligament.

The bone staple 18 preferably comprises a bone staple of the type disclosed in more detail in International publication WO 91/06249.

Instead of use of autogenous tissue to form the prosthetic ligament 15, the prosthetic ligament may comprise a synthetic device e.g of the type disclosed in more detail in EP 126520. Alternatively, the fixing device 16 of the invention may be used to secure one end of another type of flexible implantable elongate device than a ligament, e.g. a suture of the type used with patellar tendon bone block grafts.

## Claims

1. An implantable fixing device (16) for securing one end of a flexible elongate element (15) such as a prosthetic ligament which is taken through first and second bone tunnels (13, 14) formed in adjoining bones (11, 12) of a bone joint (10) said fixing device being intended to seat itself on the surface of the bone surrounding an entrance (19) to one of the bone tunnels (13) and said device comprising:
a seating device (20) comprising a collar which is shaped so as to seat at least partly on said surrounding bone surface; and
a cradle (22) which is pivotally mounted on said collar, said cradle being U shaped having a base around which the ligament can be looped and a pair of parallel legs connected to the base and which are pivotally mounted at their free ends on said collar;
wherein the cradle extends away from the seating device (20) to be located in the first bone tunnel (13), said cradle serving to hold one end of the ligament (15) and being capable of adjusting its position relative to the seating device (20), if necessary, so that the seating device can apply substantially uniform bearing pressure via an annular bearing surface (20a) while the ligament (15) can extend along the bone tunnels (13, 14) without coming into engagement with the walls of the tunnels (13, 14).

2. An implantable fixing device according to claim 1, in which the free ends of the legs (24) have lugs (25) which can seat each on a respect one of a pair of diametrically opposed seating recesses (26) formed in the collar (20).

3. An implantable fixing device according to claim 2, in which the lugs are out-turned lugs (25) which allow the U-shaped cradle (22) to be mounted on the collar (20) by inward deformation of the free ends of the legs (24), or by a relative slidable movement of the base (23) and the legs (24) through the collar until the lugs (25) become seated on the respective seating recesses (26).

4. An implantable fixing device according to any one of claims 1 to 3, in combination with said flexible elongate element, in which said elongate element (25) comprises a prosthetic ligament or a suture.

5. An implantable fixing device according to claim 4, in which the elongate element comprises a prosthetic ligament (15) made of woven implantable material, or made from autogenous tissue derived from the patient.

6. A prosthetic ligament fixation system comprising:
a prosthetic ligament;
a first fixing device as claimed in claim 1 for holding one end of the ligament at or near to one end of the first bone tunnel; and
a second fixing device for holding the opposite end of the ligament at or near to one end of the second bone tunnel.

## Patentansprüche

1. Implantierbare Befestigungsvorrichtung (16) zur sicheren Befestigung eines Endes eines flexiblen, länglichen Elements (15) wie z. B. eines künstlichen Bandes, das durch einen ersten und einen zweiten Knochentunnel (13, 14), die in aneinanderstoßenden Knochen (11, 12) eines Knochengelenks (10) gebildet wurden, geführt wird, wobei diese Befestigungsvorrichtung dazu vorgesehen ist, auf der Knochenfläche zu sitzen, die einen Eingang (19) zu einem der Knochentunnel (13) umgibt, und wobei diese Vorrichtung aus
- einer Sitzvorrichtung (20) mit einem Bundring, der so geformt ist, daß er zumindest teilweise auf der genannten umgebenden Knochenfläche sitzt, und
- einem Aufhängungsbügel (22), der drehbar an dem genannten Bundring angebracht ist, wobei dieser Aufhängungsbügel U-förmig mit einer Basis, um die das Band geschlungen werden kann, und einem Paar mit der Basis verbundener, parallel verlaufender Schenkel, die mit ihren freien Enden drehbar an dem genannten Bundring angebracht sind,
besteht, wobei sich der Aufhängungsbügel von der Sitzvorrichtung (20) weg erstreckt und in dem ersten Knochentunnel (13) angeordnet ist, wobei dieser Aufhängungsbügel dazu dient, ein Ende des Bandes (15) zu halten, und geeignet ist, ggf. seine Stellung gegenüber der Sitzvorrichtung (20) anzupassen, so daß die Sitzvorrichtung über eine ringförmige Auflagefläche (20a) einen im wesentlichen gleichförmigen Auflagedruck ausüben kann, während das Band (15) sich entlang der Knochentunnel (13, 14) erstrecken kann, ohne mit den Wänden der Tunnel (13, 14) in Eingriff zu kommen.

2. Implantierbare Befestigungsvorrichtung nach Anspruch 1, bei der die freien Enden der Schenkel (24) Nasen (25) aufweisen, die jeweils in einer von zwei einander diametral gegenüberliegenden Aufnahmevertiefungen (26), die in dem Bundring (20) ausgeführt sind, sitzen können.

3. Implantierbare Befestigungsvorrichtung nach Anspruch 2, bei der die Nasen nach außen weisende Nasen (25) sind, mittels derer der U-förmige Aufhängungsbügel (22) unter Einwärtsbiegen der freien Enden der Schenkel (24) an dem Bundring (20) angebracht werden kann, oder auch durch eine Gleitbewegung der Basis (23) und der Schenkel (24) durch den Bundring, bis die Nasen (25) in die jeweiligen Aufnahmevertiefungen (26) zu liegen kommen.

4. Implantierbare Befestigungsvorrichtung nach einem der Ansprüche 1 bis 3 in Kombination mit dem genannten flexiblen, länglichen Element (15), wobei das genannte längliche Element aus einem künstlichen Band oder einem Nahtmaterial besteht.

5. Implantierbare Befestigungsvorrichtung nach Anspruch 4, bei der das längliche Element aus einem künstlichen Band (15) aus gewobenem, implantierbarem Stoff oder aus autogenuinem Gewebe, das dem Patienten entnommen wird, besteht.

6. Künstliches Bandbefestigungssystem, bestehend aus:
- einem künstlichen Band,
- einer ersten Befestigungsvorrichtung gemäß Anspruch 1, die ein Endes des Bandes an oder nahe bei einem Ende des ersten Knochentunnels (13) festhält, und
- einer zweiten Befestigungsvorrichtung, die das andere Ende des Bandes an oder nahe bei einem Ende des zweiten Knochentunnels (14) festhält.

## Revendications

1. Dispositif de fixation implantable (16) destiné à fixer une extrémité d'un élément allongé flexible (15), tel qu'une prothèse de ligament introduite à travers des premier et un deuxième canaux osseux (13, 14) formés dans des os adjacents (11, 12) d'une articulation osseuse (10), ce dispositif de fixation étant destiné à s'adapter sur la surface de l'os qui entoure une entrée (19) de l'un des canaux osseux (13), et ce dispositif comprenant :
un dispositif d'ajustement (20) comprenant une bague dont la forme est conçue pour s adapter, au moins partiellement, sur la surface de l'os qui l'entoure ; et
un arceau (22) qui est monté pivotant sur la bague, cet arceau présentant une forme de U et comportant une base autour de laquelle le ligament peut être enroulé, et une paire de jambes parallèles reliées à la base et qui sont montées pivotantes, à leurs extrémités libres, sur la bague ;
dans lequel l'arceau est orienté à l'opposé du dispositif d'ajustement (20) destiné à être placé dans le premier canal osseux (13), cet arceau servant à maintenir une extrémité du ligament (15) et étant susceptible de régler sa position par rapport au dispositif d'ajustement (20), si nécessaire, de telle sorte que le dispositif d'ajustement puisse appliquer une pression d'appui sensiblement uniforme, par l'intermédiaire d'une surface d'appui annulaire (20a), tandis que le ligament (15) peut s'étendre le long des canaux osseux (13, 14) sans être en contact avec les parois des canaux (13, 14).

2. Dispositif de fixation implantable selon la revendication 1, dans lequel les extrémités libres des jambes (24) comportent des ergots (25) qui peuvent reposer, chacun, sur l'un des évidements formant logements (26), faisant partie d'une paire d'évidements formant logements diamétralement opposés, formés dans la bague (20).

3. Dispositif de fixation implantable selon la revendication 2, dans lequel les ergots sont des ergots (25) tournés vers l'extérieur, qui permettent à l'arceau (22) en forme de U d'être monté sur la bague (20) par déformation vers l'intérieur des extrémités libres des jambes (24), ou par un mouvement glissant relatif de la base (23) et des jambes (24) à travers la bague jusqu'à ce que les ergots (25) viennent s'adapter sur les évidements formant logements (26) respectifs.

4. Dispositif de fixation implantable selon l'une quelconque des revendications 1 à 3, en combinaison avec l'élément allongé flexible, dans lequel l'élément allongé (25) comprend une prothèse de ligament ou une suture.

5. Dispositif de fixation implantable selon la revendication 4, dans lequel l'élément allongé comprend une prothèse de ligament (15) constitué en matériau implantable tissé, ou constitué en tissu autogène prélevé sur le patient.

6. Système de fixation de prothèse de ligament, comprenant :
une prothèse de ligament ;
un premier dispositif de fixation selon la revendication 1, destiné à maintenir une extrémité du ligament à une extrémité du premier canal osseux, ou à proximité de celui-ci ; et
un deuxième dispositif de fixation destiné à maintenir l'extrémité opposée du ligament à une extrémité du deuxième canal osseux, ou à proximité de celui-ci.
